# EUROPÄISCHE PATENTSCHRIFT

(11) **EP 2 440 135 B1**
(45) Veröffentlichungstag und Bekanntmachung des Hinweises auf die Patenterteilung: **17.07.2013**
(21) Anmeldenummer: 10724844.5
(22) Anmeldetag: 09.06.2010
(51) Int. Cl.: A61B 5/15, A61B 17/34, A61M 25/00, B21C 37/08, B21G 1/08

(54) **MIKRONADEL UND VERFAHREN ZU DEREN HERSTELLUNG**
MICRONEEDLE AND METHOD FOR ITS MANUFACTURE
MICRO-AIGUILLE ET PROCEDE DESTINE A SA FABRICATION

(30) Priorität: 10.06.2009 EP 09162464
(43) Veröffentlichungstag der Anmeldung: 18.04.2012
(73) Patentinhaber: Roche Diagnostics GmbH, 68305 Mannheim (DE); F.Hoffmann-La Roche AG, 4070 Basel (CH)
(72) Erfinder: HAAR, Hans-Peter, 69168 Wiesloch (DE)
(74) Vertreter: Pfiz, Thomas
(86) Internationale Anmeldenummer: PCT/EP2010/058091
(87) Internationale Veröffentlichungsnummer: WO 2010/142728

(56) Entgegenhaltungen:
- EP-A- 1 323 483
- EP-A- 1 692 999
- DE-B1- 1 477 025
- US-A- 4 785 868
- US-A1- 2003 009 113

## Beschreibung

Die Erfindung betrifft ein Verfahren zur Herstellung einer in Körpergewebe einstechbaren Mikronadel, bei welchem eine Nadelspitze und ein vorzugsweise kapillarer Sammelkanal mit einem an der Nadelspitze ausgebildeten distalen Einlass für Köperflüssigkeit geformt wird, wobei ein Vorformling aus einem Flachmaterial vorgefertigt wird. Die Erfindung betrifft weiter eine solchermaßen hergestellte Mikronadel.

Aus der WO 2008/122541 ist ein gattungsgemäßes Verfahren bekannt. Dort wird vorgeschlagen, einen kapillaraktiven Sammelbereich für Körperflüssigkeit durch zwei gegeneinander gefaltete Biegeteile zu definieren. Damit wird erreicht, dass der Benutzer nach einem Hauteinstich die Probennahme für eine Blutzuckermessung selbst nicht weiter kontrollieren muss. Zugleich wird eine herstellungstechnische Vereinfachung dahingehend erreicht, dass der Sammelbereich ohne aufwändige Materialbearbeitungsschritte geschaffen werden kann, wie sie etwa für den Materialabtrag bei einem Drahtmaterial erforderlich wären. Durch die Faltung wird allerdings eine nur halbseitig begrenzte U-förmige Kanalstruktur geschaffen, bei der die Verdunstung der Flüssigprobe während des Kapillartransports insbesondere bei mikroskopischen Entnahmemengen noch problematisch ist. Hierbei ist auch zu beachten, dass das Zeitfenster für die Entnahme und Messung nach unten begrenzt ist, um einen hinreichenden Messerfolg noch sicherzustellen.

Ein weiteres Verfahren zur Herstellung von Injektionsnadeln aus dünnen Metallplatten ist aus EP 1323483 A2 bekannt.

Ausgehend hiervon liegt der Erfindung die Aufgabe zugrunde, die im Stand der Technik bekannten Verfahren und Vorrichtungen weiter zu verbessern und insbesondere für die Massenfertigung von analytischen Verbrauchsmitteln eine einfache Herstellbarkeit bei gleichzeitig hoher Gebrauchssicherheit zu gewährleisten.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, durch die Umformung von fertigungstechnisch vorteilhaften Flachteilen eine für den Einsatzzweck günstige 3D-Struktur zu schaffen. Dementsprechend wird erfindungsgemäß vorgeschlagen, dass zumindest ein Teil des Vorformlings zu einer Rohrstruktur umgeformt wird, so dass der Sammelkanal im Bereich der Rohrstruktur zumindest im Wesentlichen im Querschnitt ringförmig bzw. zylinderförmig geschlossen wird. Auf diese Weise wird ein besonders günstiges Verhältnis von Flüssigvolumen zu freier Oberfläche geschaffen, so dass die Verdunstung während der Probenverarbeitung deutlich reduziert werden kann. Durch die Rohrstruktur wird ggf. mit Ausnahme eines für die Verdunstung unbedeutenden Schließspalts eine umlaufende Begrenzung geschaffen, die auch für die Erhöhung der Kapillarität von Vorteil ist.

Vorteilhafterweise wird die Rohrstruktur unter Ausbildung eines längs durchlaufenden Schließspalts ringförmig gebogen, so dass ein zylindrischer Sammelkanal geschaffen wird. Hierfür ist es günstig, wenn zwei voneinander abgewandte Randkanten des Vorformlings auf Stoss zusammengebracht werden, wobei ggf. die Randkanten zumindest punktweise durch Verbindungsmittel, insbesondere durch Laserschweißstellen verbunden werden können.

Eine herstellungstechnische Vereinfachung ergibt sich dadurch, dass der Vorformling durch einen Formstempel in ein halboffenes Formnest eines Formwerkzeugs eingedrückt wird, wobei das Formnest eine Teilkontur der zu fertigenden Rohrstruktur definiert. Anschließend kann die Rohrstruktur durch einen auf freie Randkanten des Vorformlings aufgedrückten Schließstempel ausgeformt werden.

Alternativ ist es auch möglich, dass der Vorformling durch einen Ziehstein bzw. eine Matrize hindurchgezogen wird, wobei der Ziehstein eine die Kontur der Rohrstruktur definierende Öffnung aufweist.

Denkbar ist es auch, dass der Vorformling aus einer Kunststofffolie gebildet und durch Thermoformen in die Rohrstruktur umgewandelt wird.

Zur Kopplung mit einer automatischen Stech- und Messeinrichtung sind an dem Vorformlingals Haltestruktur proximal abstehenden Haltearme angeformt.

Eine weitere vorteilhafte Ausgestaltung sieht vor, dass eine Mehrzahl von Vorformlingen als zusammenhängender Verbund vorzugsweise durch Ätzen oder Laserschneiden vorstrukturiert werden.

Im Sinne einer erhöhten Systemintegration ist es weiterhin von Vorteil, wenn der Sammelkanal an einem von der Nadelspitze entfernten proximalen Auslass mit einem auf einen Inhaltsstoff der Körperflüssigkeit ansprechenden analytischen Testelement verbunden wird, so dass ein "One-Step-Handling" insbesondere für die Blutzuckerbestimmung ermöglicht wird. Vorteilhafterweise wird an einem proximalen Auslass des Sammelkanals ein die aufgenommene Körperflüssigkeit flächig verteilendes Spreitelement bzw. eine Spreitmembran angebracht, so dass eine Vergrößerung der zugänglichen Messfläche erreicht wird. Vorformling ist mittels der Haltestruktur mit einem der analytischen Testelement und/oder Lichtleiter tragenden Adapter vorzugsweise durch eine Steckverbindung verbunden.

Für eine verbesserte Anpassung an die Testgeometrie ist es günstig, wenn die Rohrstruktur mit einer von der Kreisform abweichenden, insbesondere elliptischen proximalen Öffnung versehen wird. In diesem Zusammenhang ist es auch denkbar, dass die Rohrstruktur insbesondere durch Faltlinien in dem Vorformling mit einer mehreckigen Kontur gebildet wird.

Um einen möglichst schmerzarmen Einstich zu ermöglichen, kann die Nadelspitze durch Ätzen, Schneiden oder Schleifen an dem Vorformling ausgebildet und/oder nach der Ausbildung der Rohrstruktur ausgearbeitet werden.

Für eine weiter verbesserte Flüssigkeitsaufnahme ist es vorteilhaft, wenn zumindest auf der Innenseite der Rohrstruktur eine hydrophile Schicht aufgebracht wird.

Vorteilhafterweise wird der Sammelkanal als kapillare Fließstrecke zwischen dem distalen Einlass und einer proximalen Mündung ausgebildet, so dass die aus dem Körpergewebe gewonnene Körperflüssigkeit von dem Einlass von der Nadelspitze weg zu der Mündung bzw. dem Auslass kapillaraktiv transportiert wird. Dabei ist es auch vorteilhaft, wenn der Sammelkanal eine Durchmesser im Bereich zwischen 50 bis 500 µm, vorzugsweise zwischen 100 bis 200 µm aufweist.

Gegenstand der Erfindung ist auch eine Mikronadel mit einer aus einem flachen Vorformling gebildeten und zumindest im Wesentlichen umlaufend geschlossenen Rohrstruktur gemäβ Anspruch 15.

Im Folgenden wird die Erfindung anhand der in der Zeichnung schematisch dargestellten Ausführungsbeispiele näher erläutert. Es zeigen
- Fig. 1: eine Mikronadel mit Sensoreinheit als disposibler Microsampler zur Blutzuckerbestimmung in perspektivischer Ansicht;
- Fig. 2: die Mikronadel nach Fig. 1;
- Fig. 3: einen Abschnitt der Sensoreinheit nach Fig. 1 mit integrierten Lichtleitern;
- Fig. 4: die Sensoreinheit nach Fig. 1 mit stirnseitigem Testelement;
- Fig. 5: einen Verbund von Flachformteilen als Vorformlinge von Mikronadeln in der Draufsicht;
- Fig. 6 und 7: ein Formwerkzeug zur Umformung der Vorformlinge zu Mikronadeln gemäß Fig. 2 in schaubildlicher Darstellung.

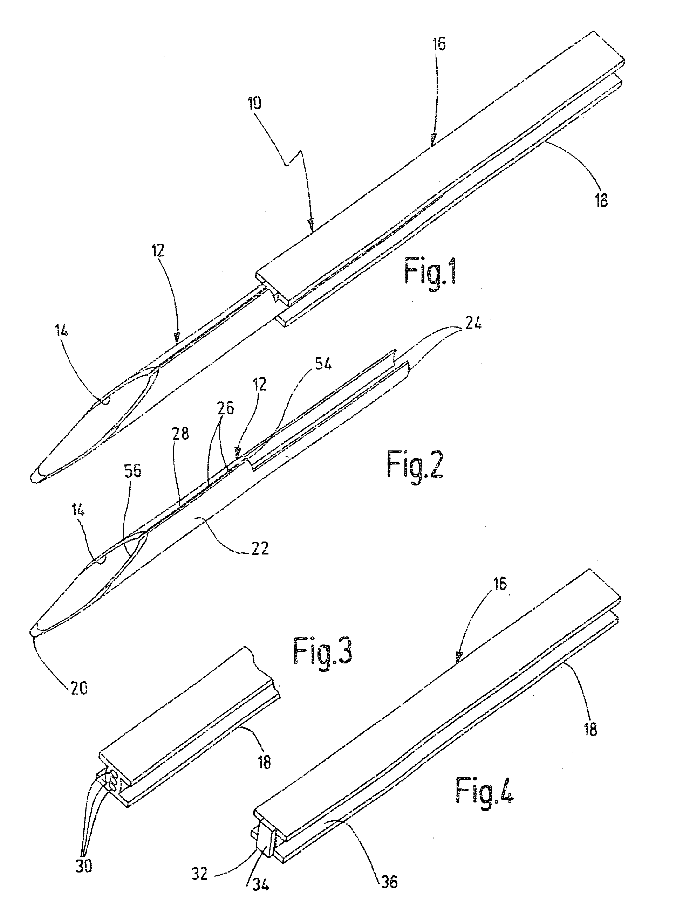

Die in Fig. 1 dargestellten Microsampler 10 lassen sich als Einmalartikel für eine Blutzuckermessung in einem dafür ausgebildeten Handgerät einsetzen, um eine mikroskopische Blutprobe zu gewinnen und vor Ort zu analysieren. Zu diesem Zweck umfassen die Microsampler 10 eine in die Haut bzw. Körpergewebe einstechbare Mikronadel 12, einen daran ausgebildeten Sammelkanal 14 zur Aufnahme von durch den Einstich erhaltener Körperflüssigkeit (Blut und/oder Gewebeflüssigkeit) über einen distalen Einlass und eine Sensoreinheit 16 für eine unmittelbare optische Probenvermessung. Die Sensoreinheit 16 weist einen mit einer Messeinrichtung koppelbaren Adapter 18 auf, wie es aus der WO 2008/122541 hervorgeht, auf die in diesem Zusammenhang Bezug genommen wird.

Wie am besten aus Fig. 2 ersichtlich, besitzt die Mikronadel 12 eine spitz zulaufende, abgeflachte distale Nadelspitze 20 als Stechorgan, eine kapillare Rohrstruktur 22 zur umlaufenden Begrenzung des Sammelkanals 14 und zwei proximal abstehende Haltearme 24 zum Aufstecken auf den Adapter 18. Die Rohrstruktur 22 ist im Querschnitt ringförmig bzw. in Längsrichtung gesehen zylindrisch gebogen, wobei die Randkanten 26 unter Begrenzung eines durchgehenden engen Schließspalts 28 auf Stoss zusammengeführt sind. Grundsätzlich ist es möglich, dass der Schließspalt 28 durch Laserschweißen zumindest punktweise überbrückt wird, so dass die Strukturstabilität noch weiter erhöht wird.

Fig. 3 zeigt den distalen Endabschnitt des Adapters 18 mit drei parallel verlaufenden integrierten Lichtleitern 30 für eine reflektometrische optische Messung. Zu diesem Zweck ist auf die distalen Lichtleiterenden ein Testelement 32 aufgebracht, wie es in Fig. 4 dargestellt ist. Zur Steckverbindung mit der Mikronadel 12 lassen sich deren Haltearme 24 in seitliche Aufnahmenuten 36 des Adapters 18 einführen. Das Testelement 32 ist somit am proximalen Ende der durch den Sammelkanal 14 gebildeten Fließstrecke angeordnet. Der zylindrische Sammelkanal 14 gibt die Flüssigprobe nur an seinem Ende ab und sollte dazu einen lichten Durchmesser zweckmäßig im Bereich zwischen 100 bis 200 µm aufweisen. Im Messzustand steht das Testelement 32 über eine vorderseitige Nachweisschicht 34 in fluidischem Kontakt mit der proximalen Mündung des Sammelkanals 14, während über die Lichtleiter 30 eine rückseitige optische Erfassung einer Farbänderung möglich ist, die auf einer Reaktion mit Blutglukose beruht. Für eine sichere endständige Benetzung ist es wichtig, dass die Blutflüssigkeit ohne Verdunstungsverluste transportiert werden kann, was durch den im Wesentlichen seitlich geschlossenen Sammelkanal 14 gewährleistet ist.

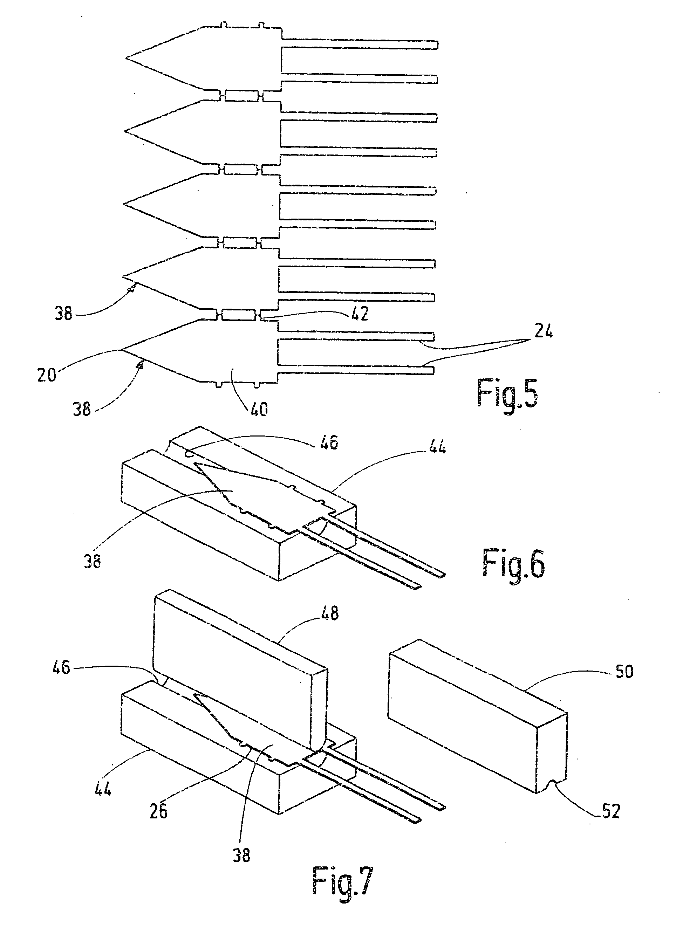

Um eine kostengünstige Massenfertigung zu ermöglichen, wird die Hohlstruktur aus einem Flachmaterial erzeugt, wie es in Fig. 5 bis 7 veranschaulicht ist.

Fig. 5 zeigt einen Verbund von Flachlanzetten als Vorformlinge 38, die beispielsweise aus einer Edelstahlfolie durch einen Ätzprozess oder Laserschneiden gebildet werden können, so dass die Nadelspitze 20, die Wandung 40 der Rohrstruktur 22 und die Haltearme 24 bereits in der Materialebene vorstrukturiert sind. Die Vorformlinge 38 können dabei noch über Materialbrücken 42 verbunden bleiben.

In einem nachfolgenden Umformprozess werden die Vorformlinge 38 vereinzelt auf einem Formwerkzeug 44 positioniert, wie es in Fig. 6 veranschaulicht ist. Das Formwerkzeug 44 weist ein halboffenes, etwa U-förmiges Formnest 46 auf, das eine Teilkontur der zu fertigenden Rohrstruktur definiert.

Die Biegeumformung erfolgt dann durch Stempel 48, 50 gemäß Fig. 7. Zunächst wird der Vorformling 38 durch den Formstempel 48 nach unten in das Formnest 46 eingedrückt, so dass die untere Halbseite der Rohrstruktur 22 geprägt wird. Sodann wird der Schließstempel 50 auf die nach oben überstehenden Randkanten 26 aufgedrückt, um die obere Halbseite der Rohrstruktur 22 entsprechend der Stirnkontur 52 des Schließstempels 50 ringförmig zu schließen. Das auf diese Weise erzeugte Formteil entspricht dann der Mikronadel gemäß Fig. 2. Gegebenenfalls kann die Nadelspitze 20 noch durch eine Nachbearbeitung, insbesondere durch einen Anschliff geschärft werden.

Die Rohrstruktur 22 kann einen durchgehenden kreisförmigen Querschnitt aufweisen. Zur Anpassung an die Rechteckform des Testelements 32 ist es auch möglich, den proximalen Bereich abzuflachen, so dass eine ovale bzw. elliptische Mündungsöffnung 54 erreicht wird. Durch die Wandverengung wird auch eine in Transportrichtung zunehmende Kapillarität erreicht, die eine sichere Benetzung des Testelements 32 begünstigt. In diesem Zusammenhang ist es auch günstig, wenn die Innenseite der Rohrstruktur 22 mit einer hydrophilen Schicht 56 versehen ist, die bereits im Zuge der Flachmaterialverarbeitung als Fließstrecke für die Körperflüssigkeit aufgebracht werden kann.

## Patentansprüche

1. Verfahren zur Herstellung einer in Körpergewebe einstechbaren Mikronadel (12), bei welchem eine Nadelspitze (20) und ein vorzugsweise kapillarer Sammelkanal (14) mit einem an der Nadelspitze (20) ausgebildeten distalen Einlass für Köperflüssigkeit geformt wird, wobei
a) ein Vorformling (38) aus einem Flachmaterial vorgefertigt wird,
b) an dem Vorformling (38) proximal abstehende Haltearme als Haltestruktur (24) angeformt werden,
c) zumindest ein Teil des Vorformlings (38) zu einer Rohrstruktur (22) umgeformt wird, so dass der Sammelkanal (14) im Bereich der Rohrstruktur (22) zumindest im Wesentlichen im Querschnitt ringförmig geschlossen wird, **dadurch gekennzeichnet, dass**
d) der Vorformling (38) mittels der Haltestruktur (24) mit einem ein analytisches Testelement (32) und/oder Lichtleiter (30) tragenden Adapter (18) verbunden wird.

2. Verfahren nach Anspruch 1, **dadurch gekennzeichnet, dass** die Rohrstruktur (22) unter Ausbildung eines längs durchlaufenden Schließspalts (28) im Querschnitt ringförmig gebogen wird.

3. Verfahren nach Anspruch 1 oder 2, **dadurch gekennzeichnet, dass** zwei voneinander abgewandte Randkanten (26) des Vorformlings (38) auf Stoss zusammengebracht werden, und dass die Randkanten (26) zumindest punktweise durch Verbindungsmittel, insbesondere durch Laserschweißstellen verbunden werden.

4. Verfahren nach einem der Ansprüche 1 bis 3, **dadurch gekennzeichnet, dass** der Vorformling (38) durch einen Formstempel (48) in ein halboffenes Formnest (46) eines Formwerkzeugs (44) eingedrückt wird, wobei das Formnest (46) eine Teilkontur der zu fertigenden Rohrstruktur (22) definiert.

5. Verfahren nach einem der Ansprüche 1 bis 4, **dadurch gekennzeichnet, dass** die Rohrstruktur (22) durch einen auf freie Randkanten (26) des Vorformlings (38) aufgedrückten Schließstempel (50) ausgeformt wird.

6. Verfahren nach einem der Ansprüche 1 bis 3, **dadurch gekennzeichnet, dass** der Vorformling (38) durch einen Ziehstein hindurchgezogen wird, wobei der Ziehstein eine die Kontur der Rohrstruktur (22) definierende Öffnung aufweist.

7. Verfahren nach einem der Ansprüche 1 bis 3, **dadurch gekennzeichnet, dass** der Vorformling (38) aus einer Kunststofffolie gebildet und durch Thermoformen in die Rohrstruktur (22) umgewandelt wird.

8. Verfahren nach einem der Ansprüche 1 bis 7, **dadurch gekennzeichnet, dass** eine Mehrzahl von Vorformlingen (38) als zusammenhängender Verbund vorzugsweise durch Ätzen oder Laserschneiden vorstrukturiert werden.

9. Verfahren nach einem der Ansprüche 1 bis 8, **dadurch gekennzeichnet, dass** der Sammelkanal (14) an einem proximalen Auslass mit einem auf einen Inhaltsstoff der Körperflüssigkeit ansprechenden analytischen Testelement (32) verbunden wird.

10. Verfahren nach einem der Ansprüche 1 bis 9, **dadurch gekennzeichnet, dass** an einem proximalen Auslass des Sammelkanals (14) ein die aufgenommene Körperflüssigkeit flächig verteilendes Spreitelement angebracht wird.

11. Verfahren nach einem der Ansprüche 1 bis 10, **dadurch gekennzeichnet, dass** der Vorformling (38) mittels der Haltestruktur (24) mit einem ein analytisches Testelement (32) und/oder Lichtleiter (30) tragenden Adapter (18) durch eine Steckverbindung verbunden wird.

12. Verfahren nach einem der Ansprüche 1 bis 11, **dadurch gekennzeichnet, dass** die Rohrstruktur (22) mit einer von der Kreisform abweichenden, insbesondere elliptischen proximalen Öffnung (54) versehen wird.

13. Verfahren nach einem der Ansprüche 1 bis 12, **dadurch gekennzeichnet, dass** die Nadelspitze (20) durch Ätzen, Schneiden oder Schleifen an dem Vorformling (38) ausgebildet und/oder nach der Ausbildung der Rohrstruktur (22) ausgearbeitet wird.

14. Verfahren nach einem der Ansprüche 1 bis 13, **dadurch gekennzeichnet, dass** der Sammelkanal (14) als kapillare Fließstrecke zwischen dem distalen Einlass und einem proximalen Auslass ausgebildet wird, so dass die Körperflüssigkeit von dem Einlass zu dem Auslass kapillaraktiv transportiert wird.

15. Mikronadel hergestellt nach dem Verfahren gemäß einem der vorhergehenden Ansprüche, mit einer zumindest im Wesentlichen umlaufend geschlossenen Rohrstruktur (22), **dadurch gekennzeichnet, dass** die Rohrstruktur (22) mit proximal abstehenden Haltearmen als Haltestruktur (24) versehen ist und mittels der Haltestruktur (24) mit einem ein analytisches Testelement (32) und/oder Lichtleiter (30) tragenden Adapter (18) verbunden ist.

## Claims

1. Process for producing a microneedle (12) that can be inserted into body tissue in which a needle tip (20) and a preferably capillary collecting channel (14) having a distal inlet for body fluid formed at the needle tip (20) are formed, wherein
a) a preform (38) is prefabricated of a flat material,
b) proximally projecting holding arms are formed on the preform (38) as a holding structure (24),
c) at least a part of the preform (38) is shaped into a tubular structure (22) so that the collecting channel (14) is at least substantially annularly closed in the cross-section in the area of the tubular structure (22), **characterized in that**
d) the preform (38) is connected by means of the holding structure (24) to an adapter (18) carrying an analytical test element (32) and/or light guides (30).

2. Process according to claim 1, **characterized in that** the cross-section of the tubular structure (22) is bent annularly while forming a longitudinal continuous closing gap (28).

3. Process according to claim 1 or 2, **characterized in that** two outer edges (26) of the preform (38) which face away from one another are abutted and the outer edges (26) are joined at least point-like by joining means and in particular by laser welding spots.

4. Process according to one of the claims 1 to 3, **characterized in that** the preform (38) is pressed by a form punch (48) into a semi-open mould cavity (46) of a forming tool (44) where the mould cavity (46) defines a partial contour of the tubular structure (22) to be manufactured.

5. Process according to one of the claims 1 to 4, **characterized in that** the tubular structure (22) is shaped by a closing punch (50) pressed onto the free outer edges (26) of the preform (38).

6. Process according to one of the claims 1 to 3, **characterized in that** the preform (38) is pulled through a drawing die, where the drawing die has an opening defining the contour of the tubular structure (22).

7. Process according to one of the claims 1 to 3, **characterized in that** the preform (38) is formed from a plastic foil and is converted into the tubular structure (22) by thermoforming.

8. Process according to one of the claims 1 to 7, **characterized in that** a plurality of preforms (38) are prestructured as a contiguous composite preferably by etching or laser cutting.

9. Process according to one of the claims 1 to 8, **characterized in that** the collecting channel (14) is connected at a proximal outlet to an analytical test element (32) that reacts to a component of the body fluid.

10. Process according to one of the claims 1 to 9, **characterized in that** a spreading element which distributes the collected body fluid two-dimensionally is attached to a proximal outlet of the collecting channel (14).

11. Process according to one of the claims 1 to 10, **characterized in that** the preform (38) is connected by means of the holding structure (24) to an adapter (18) carrying an analytical test element (32) and/or light guides (30) by means of a plug connection.

12. Process according to one of the claims 1 to 11, **characterized in that** the tubular structure (22) is provided with an in particular clliptical proximal opening (54) that deviates from a circular shape.

13. Process according to one of the claims 1 to 12, **characterized in that** the needle tip (20) is formed on the preform (38) by etching, cutting or grinding and/or made after formation of the tubular structure (22).

14. Process according to one of the claims 1 to 13, **characterized in that** collecting channel (14) is formed as a capillary flow path between the distal inlet and a proximal outlet so that body fluid is transported by capillary action from the inlet to the outlet.

15. Microneedle produced by the process according to one of the previous claims, with an at least essentially circumferentially closed tubular structure (22), **characterized in that** the tubular structure (22) is provided with proximally projecting holding arms s a holding structure (24) and is connected by means of the holding structure (24) to an adapter (18) carrying an analytical test element (32) and/or light guides (30).

## Revendications

1. Procédé de fabrication d'une micro-aiguille (12) pouvant être introduite dans un tissu corporel, consistant à former une pointe d'aiguille (20) et un canal collecteur (14) de préférence capillaire muni d'une entrée distale formée sur la pointe d'aiguille (20) pour un fluide corporel, dans lequel
a) on réalise au préalable une ébauche (38) en un matériau plat,
b) on forme sur l'ébauche (38), comme structure de support (24), des bras de support faisant saillie du côté proximal,
c) on transforme au moins une partie de l'ébauche (38) en une structure tubulaire (22), de telle sorte que le canal collecteur (14), dans la région de la structure tubulaire (22), soit fermé au moins sensiblement en forme d'anneau en section transversale, **caractérisé en ce que**
d) on relie l'ébauche (38), au moyen de la structure de support (24), à un adaptateur (18) portant un élément de test analytique (32) et/ou un guide de lumière (30).

2. Procédé selon la revendication 1, **caractérisé en ce que** la structure tubulaire (22) est courbée en forme d'anneau en section transversale, avec formation d'une fente de fermeture (28) continue longitudinale.

3. Procédé selon la revendication 1 ou 2, **caractérisé en ce que** deux bords périphériques (26) opposés l'un à l'autre de l'ébauche (38) sont amenés en aboutement, et **en ce que** les bords périphériques (26) sont assemblés, au moins par points, par des moyens d'assemblage, en particulier par des soudures laser.

4. Procédé selon l'une des revendications 1 à 3, **caractérisé en ce que** l'ébauche (38) est enfoncée, par un poinçon de formage (48), dans une cavité de formage semi-ouverte (46) d'un outil de formage (44), ladite cavité de formage (46) définissant un contour partiel de la structure tubulaire (22) à fabriquer.

5. Procédé selon l'une des revendications 1 à 4, **caractérisé en ce que** la structure tubulaire (22) est formée par un poinçon de fermeture (50) appliqué sur les bords périphériques libres (26) de l'ébauche (38).

6. Procédé selon l'une des revendications 1 à 3, **caractérisé en ce que** l'ébauche (38) est tirée à travers une filière, ladite filière comportant une ouverture définissant le contour de la structure tubulaire (22).

7. Procédé selon l'une des revendications 1 à 3, **caractérisé en ce que** l'ébauche (38) est formée à partir d'une feuille de matière plastique et transformée en structure tubulaire (22) par thermoformage.

8. Procédé selon l'une des revendications 1 à 7, **caractérisé en ce qu'**une pluralité d'ébauches (38) sont préstructurées en tant qu'ensemble cohérent, de préférence par décapage ou découpage au laser.

9. Procédé selon l'une des revendications 1 à 8, **caractérisé en ce que** le canal collecteur (14) est relié, à une sortie proximale, à un élément de test analytique (32) sensible à un composant du fluide corporel.

10. Procédé selon l'une des revendications 1 à 9, **caractérisé en ce qu'**un élément d'étalement est fixé à une sortie proximale du canal collecteur (14), qui répartit en surface le fluide corporel prélevé.

11. Procédé selon l'une des revendications 1 à 10, **caractérisé en ce que** l'ébauche (38) est relié, au moyen de la structure de support (24), à un adaptateur (18) portant un élément de test analytique (32) et/ou un guide de lumière (30), avec un connecteur.

12. Procédé selon l'une des revendications 1 à 11, **caractérisé en ce que** la structure tubulaire (22) est munie d'une ouverture proximale (54) s'écartant de la forme circulaire, en particulier de forme elliptique.

13. Procédé selon l'une des revendications 1 à 12, **caractérisé en ce que** la pointe d'aiguille (20) est formée sur l'ébauche (38) par décapage, découpage ou meulage et/ou est réalisée après la formation de la structure tubulaire (22).

14. Procédé selon l'une des revendications 1 à 13, **caractérisé en ce que** le canal collecteur (14) est réalisé comme parcours d'écoulement libre entre l'entrée distale et une sortie proximale, de telle sorte que le fluide corporel soit transporté de ladite entrée vers ladite sortie par effet capillaire.

15. Micro-aiguille fabriquée suivant le procédé selon l'une des revendications précédentes, présentant une structure tubulaire (22) au moins sensiblement fermée sur tout le pourtour, **caractérisée en ce que** la structure tubulaire (22) est pourvue, comme structure de support (24), de bras de support faisant saillie du côté proximal et est reliée, au moyen de la structure de support (24), à un adaptateur (18) portant un élément de test analytique (32) et/ou un guide de lumière (30).
